(19) Europäisches Patentamt
European Patent Office
Office européen des brevets

(11) **EP 3 917 400 B1**

(12) **EUROPEAN PATENT SPECIFICATION**

(45) Date of publication and mention
of the grant of the patent:
**20.08.2025 Bulletin 2025/34**

(21) Application number: **20708553.1**

(22) Date of filing: **28.01.2020**

(51) International Patent Classification (IPC):
*G10L 25/66* (2013.01)   *G10L 25/63* (2013.01)
*A61B 5/16* (2006.01)   *A61B 5/021* (2006.01)
*A61B 5/00* (2006.01)   *A61B 5/024* (2006.01)
*A61B 5/08* (2006.01)   *A61B 5/0533* (2021.01)
*A61B 5/11* (2006.01)   *A61B 5/01* (2006.01)

(52) Cooperative Patent Classification (CPC):
**G10L 25/63; A61B 5/02108; A61B 5/02405;**
**A61B 5/02438; A61B 5/1118; A61B 5/165;**
**A61B 5/486; A61B 5/681; A61B 5/6824;**
**A61B 5/6831; A61B 5/7264; A61B 5/7275;**
**A61B 5/7278; G10L 25/66;** A61B 5/01;   (Cont.)

(86) International application number:
**PCT/GB2020/050202**

(87) International publication number:
**WO 2020/157493 (06.08.2020 Gazette 2020/32)**

(54) **MENTAL STATE DETERMINATION METHOD AND SYSTEM**

VERFAHREN UND SYSTEM ZUR BESTIMMUNG DES MENTALEN ZUSTANDS

PROCÉDÉ ET SYSTÈME DE DÉTERMINATION D'ÉTAT MENTAL

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **28.01.2019 GB 201901158**

(43) Date of publication of application:
**08.12.2021 Bulletin 2021/49**

(73) Proprietor: **Limbic Limited**
**London EC1V 2NX (GB)**

(72) Inventors:
• SOUTHERN, Joshua
**London, Greater London EC2A 3AZ (GB)**
• HARPER, Ross
**London, Greater London EC2A 3AZ (GB)**
• DE VRIES, Sebastiaan
**London, Greater London EC2A 3AZ (GB)**

(74) Representative: **Marks & Clerk LLP**
**15 Fetter Lane**
**London EC4A 1BW (GB)**

(56) References cited:
WO-A2-2011/109716   US-A1- 2014 089 399
US-A1- 2018 032 126   US-B1- 9 566 411
US-B2- 10 068 588   US-B2- 9 031 293

• RINCON JAIME A ET AL: "Intelligent Wristbands
for the Automatic Detection of Emotional States
for the Elderly", 9 November 2018, ADVANCES IN
DATABASES AND INFORMATION SYSTEMS;
[LECTURE NOTES IN COMPUTER SCIENCE;
LECT.NOTES COMPUTER], SPRINGER
INTERNATIONAL PUBLISHING, CHAM, PAGE(S)
520 - 530, ISBN: 978-3-319-10403-4, XP047493626

(52) Cooperative Patent Classification (CPC): (Cont.)
A61B 5/0533; A61B 5/0816; A61B 5/318;
A61B 5/6804; A61B 2562/0219

**Description**

Field

The present invention relates to the determination or classification of the mental state of a user and associated confidence values for the determined mental state. More particularly, the present invention relates to the use of various sensor data to determine the mental states of users, including optical heart data, and optionally electrocardiography data for example, from wearable devices (in a variety of form-factors, for example smart watches, fitness trackers, smart rings, smart textile, headsets, or wearable patches) or other devices having sensors operable to detect relevant attributes of a user that can be used to determine the user's mental state, such as physiological attributes.

Background

Recently, wrist-worn monitoring devices have become increasingly popular, especially with users and consumers interested in exercise and physical health. Various studies into expenditures on wearable monitoring devices show that within markets such as the sporting goods market, in particular amongst those who engage in running as a hobby or sporting activity, there is a high penetration of such devices. That said, beyond these specific markets, the uptake of wrist-worn wearable devices remains low (especially in comparison with the uptake of smartphones) at around 15% of the US population and between roughly 6 and 12% of the populations of European countries.

There has been a desire to improve the capabilities of wearable devices to broaden their appeal outside of the sporting goods market, with a focus on providing general lifestyle utility. Wearable devices are being further developed to incorporate a variety of sensors including for example: optical sensors, electro-cardiogram (ECG) sensors, skin conduction/electro-dermal activity (EDA) sensors, temperature sensors, accelerometers, and/or gyroscopes.

Optical heart rate sensors are the most prevalent sensor provided on most smart watches or fitness bands. These work using a technique called photoplethysmography (PPG) to measure heart rate, which involves shining a light into the skin of the wearer and measuring the amount of light returned to the optical sensor. Typically, green light is emitted into the skin of the wearer. Usually the optical emitter in the wearable device uses two or more light emitting diodes (LEDs) to send light waves into the skin. Because of the wide range of permutations of skin tone, thickness, and morphology of users, multiple light wavelengths are typically used by the optical emitter to compensate for the optical performance across these permutations. Light returned to the optical sensor is scattered/refracted by the blood flow within the wearer's arm and the underlying physiological state of the wearer can thus be determined to a certain extent from this returned light. The optical sensor incorporates a digital signal processor (DSP) that receives the returned light data from the optical sensor and processes this data into more useful data such as for example heart rate data. By measuring changes in light absorption, it is possible to determine the heartbeat in a wearer. Algorithms process the signals output from the DSP (and potentially combine this with other data, or other data input and processes by the DSP) for use by the wearable device.

With these enhanced hardware capabilities in wearable devices, further functionality in these devices is possible.

Other sources of data and devices can be used to gather data on users that can be used to derive certain information about a user over time.

User data can be used to determine the mental state of a user, so the current mental state of a user can be detected from text, video or speech of a user for example. Mental state detection from both audio and video data relies heavily on expression, which can vary significantly across individuals and cultures and leaves room for deception. Wearable devices can provide sensor data that can enable mental state detection for a wearer.

Due to the plethora of devices now used by many people, it is thus possible to perform emotion detection and mental state detection using devices such as cameras, microphones, and data collected by mobile phones and wearable devices. It is otherwise known from US 2018/032126 A1, RINCON JAIME A ET AL: "Intelligent Wristbands for the Automatic Detection of Emotional States for the Elderly", 9 November 2018 (2018-11-09), ADVANCES IN DATABASES AND INFORMATION SYSTEMS; [LECTURE NOTES IN COMPUTER SCIENCE; LECT.NOTES COMPUTER], SPRINGER INTERNATIONAL PUBLISHING, CHAM, PAGE(S) 520 - 530, XP047493626,ISBN: 978-3-319-10403-4, and US 9 566 411 B1 to determine a mental state of a user based on photoplethysmography (PPG) time series and a trained neural network.

Summary of Invention

Aspects and/or embodiments seek to provide methods and systems for detecting (or determining) a mental state and a confidence values for the detected mental state of a user. The invention is as defined in appended independent claims 1, 11-12 and 14. Preferred embodiments are set for the dependent claims.

According to a first aspect, there is provided a method as recited in claim 1.

The mental state of a user can include any or any combination of: an emotional state; a plurality of emotional states; one or more discrete emotional states; one or more continuous emotional states; one or more discrete or continuous measures of emotion; one or more psychological states; one or more psychological states linked with mental illness; or one or more psychological states comprising any or any combination of depression, anxiety, bipolar disorder and psychosis; discrete emotions such as depression, happiness, pleasure, displeasure; and/or dimensional emotions such as arousal and valence.

Providing or having an associated confidence value for each determined mental state can provide a richer output for display to a user, or can allow the confidence value to be factored in to decision-making by either the user or a medical professional on how to act on the mental state prediction, and/or allowing low-confidence results to be flagged or ignored or not output by the learned algorithm (or model).

In many real-world scenarios, confidence can be a key factor to decision-making (e.g. in healthcare). For applications in these domains, where confidence is a key factor, it can be critical for mental state detection methods to have the capacity to describe uncertainty in their mental state detection output. By determining a measure of certainty in the model output, e.g. by determining a confidence value/values for the mental states output by the model, the decision on whether or not to accept the model's prediction can then be specified according to model confidence, rather than the output value.

Optionally the user attribute data further comprises any of or any combination of: physiological data; face imaging data; image and/or text data; speech data; electrodermal activity data; electrocardiogram data; respiration data; temperature data; gyroscope data; wearer activity data and/or accelerometer data.

In some embodiments, the type, amount or combination of sensor/user attribute data used to determine the mental state of a user can provide a substantially more accurate determined mental state. For example, should a companion device such as a mobile phone or tablet computer be connected wirelessly to a wearable device then the sensor data used to determine a user's mental state may further comprise face imaging data from the user-facing camera in the mobile phone or tablet, or image and/or text data that the user is viewing or writing on the mobile phone or tablet computer, as well as user attribute data such as physiological data obtained from the wearable device. In this example, sentiment analysis can be performed on for example the image and/or text data to extract emotional content. In another example, in addition or instead, a wearable device may comprise sensors that can provide any of or any combination of face imaging data, respiration data; temperature data; gyroscope data; wearer activity data and/or accelerometer data.

Optionally the sensors can be located on any or any combination of: a mobile phone; a computer; a wearable device; an imaging camera; an audio sensor; an audio visual device; a smartwatch; a wearable sensor; a fitness band; a smart ring, a smart textile, a headset or a wearable patch.

Providing a learned algorithm to determine the mental state of the user of a device such as a smartphone, smart watch or fitness tracker allows end-to-end prediction of a user's mental state substantially accurately from their personal devices.

Optionally, the method is for use to provide medical professionals with patient data.

Optionally, receiving user attribute data from the one or more sensors comprises receiving user attribute data from the one or more sensors substantially continuously; determining a mental state of the user from the user attribute data using a learned algorithm comprises substantially continuously determining a mental state of the user from the user attribute data using a learned algorithm; and outputting one or more determined mental states for the user comprises outputting one or more substantially continuously determined mental states for the user.

Optionally, the method can further output user attribute data corresponding to the determined mental states of the user for use by a medical professional.

Optionally, the learned algorithm is configured to determine the mental state of the user using both the user attribute data and one or more previously determined mental states of the user. In some embodiments, using the previous mental states of the user along with the user attribute data (for example user physiological data or other data about a user that can be used to determine a user mental state) can provide a substantially faster and/or more accurate determination of the mental state of the user.

Optionally, the determined mental state of the user can be associated with a location of the user and each associated mental state of the user and associated location of the user are stored in a database; optionally wherein the database is a local database or a remote database; and further optionally wherein the sensor data includes user location data.

In some embodiments, correlating the location of the user and the mental state of the user can provide for constructing a geographical map of user emotion/mental state or a geographical map of the emotions/mental states of a plurality of users.

Optionally, the learned algorithm is operable to be updated by the remote computing system; optionally wherein the learned algorithm is operable to be updated by the remote computing system using some or all of the received user attribute data and/or the determined user mental states; and/or the remote computing system is able to perform at least a portion of the step of determining the mental state of the user.

In some embodiments, updates of the model and/or performing some of the processing at a remote system can provide the advantage of improving the model and/or allowing more processing power to be used to compute the model.

Optionally, the method further comprises receiving any or any combination of: an activity level of the user; an exercise measure of the user and/or a lifestyle measure of the user over a period of time and determining a correlation between the mental state of the user and any or any combination of: the activity level of the user; the exercise measure of the user and/or the lifestyle measure of the user; optionally generating a notification for display to a user in response to the determined correlation.

In some embodiments, correlating activity/exercise/lifestyle metrics for a user can provide the advantage of being able to notify the user if activity/exercise/lifestyle is improving their mental state or that adjusting activity/exercise/lifestyle may improve their mental state based on historical data.

Optionally the mental state of a user can be communicated to a recipient.

In some embodiments, allowing a user to send information to others about their mental state can provide the advantage of allowing users to share this type of information with friends, family and/or professionals, optionally automatically. According to a further aspect, there is provided a system operable to receive one or more determined user mental states and associating the one or more user mental states with user location data determine the location at which the wearer experienced each determined mental state.

According to a further aspect, there is provided a wearable apparatus as recited in claim 14.

Providing a learned algorithm to determine the mental state of the wearer of a wearable device such as a smart watch or fitness tracker allows end-to-end prediction of a wearer's mental state substantially accurately.

In some embodiments, providing an associated confidence value can provide the advantage of providing a richer output for display to a user, factoring in to decision-making on how to act on the mental state prediction, and/or allowing low-confidence results to be flagged or ignored.

According to an aspect, there is provided an apparatus as recited in claim 12.

In some embodiments, performing updates of the model and/or performing some of the processing at a remote system can provide for iteratively improving the model and/or allowing more processing power to be used to compute the model.

Correlating the location of a user and the mental state of the user at that location can allow the construction of a geographical map overlaid with user mental state data or a geographical map of the mental states of a plurality of users.

According to another aspect, the method of the recited aspects can be performed on any device or system suitable for determining the mental state of a human. Optionally said device or system comprises a mobile device or a wearable device. Optionally said device or system comprises an imaging and/or audio recording and/or audio-visual apparatus or system. Optionally said any device or system can provide input/sensor data for determining emotion data/an mental state comprising any or any combination of: face imaging data; image and/or text data; speech data; electrodermal activity data; electrocardiogram data; photoplethysmography data; respiration data; temperature data; gyroscope data; wearer activity data and/or accelerometer data.

Correlating activity/exercise/lifestyle metrics for a user can provide the advantage of being able to notify the user if activity/exercise/lifestyle is improving their mental state or that adjusting activity/exercise/lifestyle may improve the mental state based on historical data for that user.

Allowing user to send information to others about their mental state can provide the advantage of allowing users to share this type of information with friends, family or professionals, optionally automatically.

According to a further aspect, there is provided a computer program product for providing the method of the above aspects. According to further aspects, an apparatus and/or system can be provided operable to provide equivalent features to the method aspects/embodiments set out herein.

Aspects and/or embodiments can be used in a variety of use cases, including: using emotional or mental state data gathered and/or as it is being gathered to monitor and/or diagnose mental state/illness; using emotional data or mental state data gathered and/or as it is being gathered to monitor and/or identify mood-specific characteristics; using emotional or mental state data gathered and/or as it is being gathered to determine a correlation between physical activity and mood; using emotional or mental state data gathered and/or as it is being gathered to determine a correlation between sleep and mood; using emotional or mental state data gathered and/or as it is being gathered to determine a correlation between location and mood; using emotional or mental state data gathered and/or as it is being gathered to determine a correlation between any other available data stream and mood.

Brief Description of Drawings

Embodiments will now be described, by way of example only and with reference to the accompanying drawings having like-reference numerals, in which:

Figure 1 illustrates a typical smart watch;

Figure 2 illustrates the working of an optical heart rate sensor on the example typical smart watch of Figure 1;

Figure 3 illustrates a table of sample emotion-eliciting videos that can be used during the training process for the model of the specific embodiment;

Figure 4 illustrates the structure of the model according to the specific embodiment; and

Figure 5 illustrated the probabilistic classification framework according to the model of the embodiment shown in Figure 4.

Providing measures of mental wellness using a wearable device is possible, using the sensors now typically provided on smart watches and fitness bands, and would provide the ability to monitor both individual users as well as populations and groups within populations of users.

For example, heart rate variability (HRV) is a biomarker that is straightforward to calculate using existing sensors on wearable devices and can be used to quantify physiological stress. As described above, it is possible to use sensors such as optical heartrate sensors to determine a wearer's heartbeat time series using a wearable device. More specifically, because activity in the sympathetic nervous system acts to trigger physiological changes in a wearer of a device associated with a "fight or flight" response, the wearer's heartbeat becomes more regular when this happens, thus their HRV decreases. In contrast, activity in the antagonistic parasympathetic nervous system acts to increase HRV and a wearer's heartbeat becomes less regular. Thus, it is straightforward to determine HRV using a wearable device by monitoring and tracking a wearer's heartbeat over time. It is however currently difficult to determine whether the changes in HRV that can be detected are mentally "positive", i.e. indicate eustress, or mentally "negative", i.e. indicate distress, as HRV may change in the same way for a variety of positive or negative reasons - therefore monitoring solely HRV does not provide a meaningful determination of a wearer's mental state.

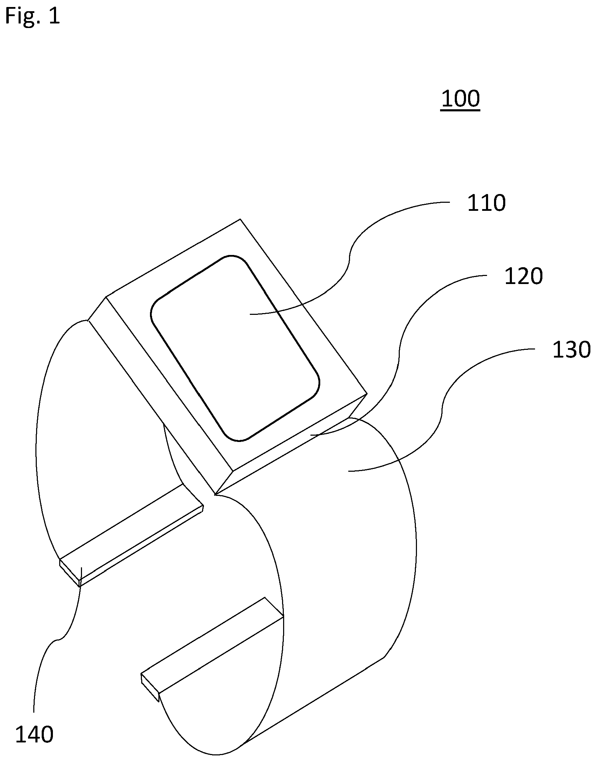

Referring to Figure 1, a typical smartwatch 100 used in the described embodiment is shown. The smartwatch 100 is provided with an optical heart rate sensor (not shown) integrated into the body 120, a display 110 that is usually a touchscreen to both display information and graphics to the wearer as well as allow control and input by a user of the device, and a strap 130 and fastener 140 to attach the device 100 to a wearer's wrist.

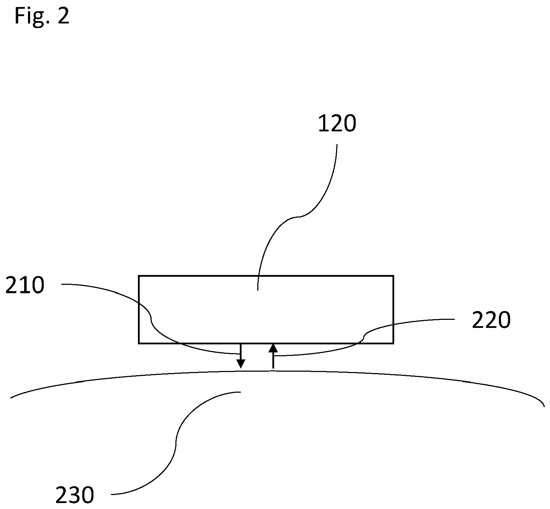

In alternative embodiments, other wearable devices in place of a smartwatch 100 can be used, including but not limited to fitness trackers, rings or smartphones. Referring to Figure 2, the optical emitter integrated into the smartwatch body 120 of Figure 1 emits light 210 into the wearer's arm 230 and then any returned light 220 is input into the optical light sensor integrated in the smartwatch body 120.

Further sensors, as outlined above, can be integrated into the smartwatch body 120 in alternative embodiments, or sensors from multiple devices can be used in further alternative embodiments.

In the present embodiment, a deep learning neural network model is trained on users with smartwatches 100. The input data to the model from the smartwatches 100 is the inter-beat intervals (IBI) extracted from the photoplethysmography (PPG) time series.

In other embodiments, other input data can be used instead, or in combination with the IBI from the PPG time series. For example, but not limited to, any or any combination of: electrodermal activity data; electrocardiogram data; respiration data and skin temperature data can be used in combination with the IBI from the PPG time series. Alternatively, other data from the PPG time series can be used in combination with or instead of the IBI from the PPG time series or the other mentioned data.

The model uses a deep learning architecture to provide an end-to-end computation of the mental state of a wearer of the smartwatch 100 directly based on this input data. Once the model is trained, a trained model is produced that can be deployed on smartwatches 100 that works without needing further training and without needing to communicate with remote servers to update the model or perform off-device computation.

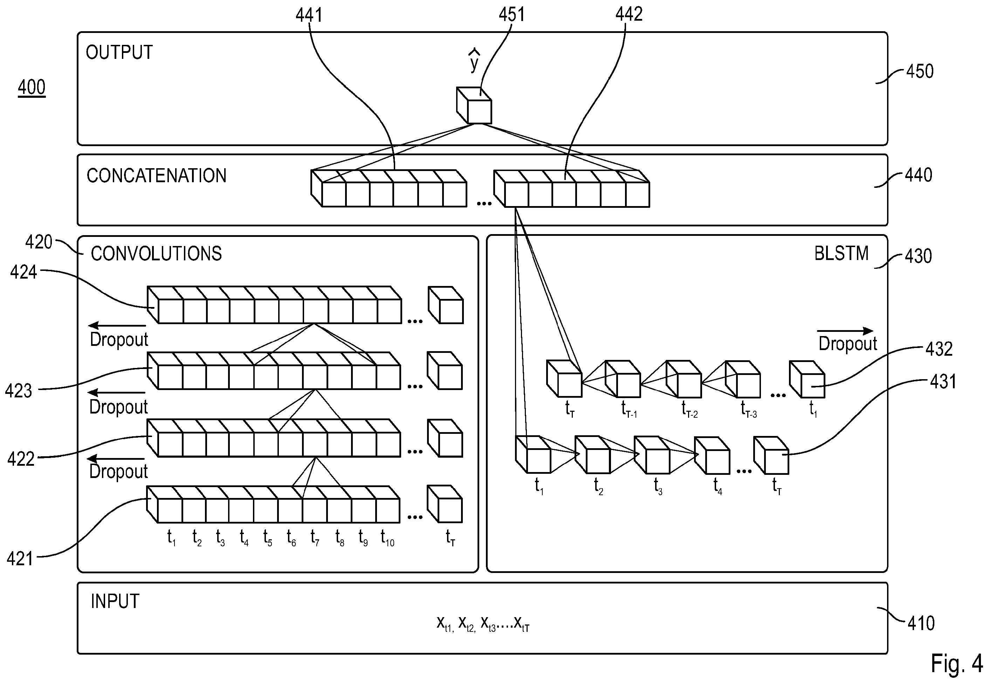

Referring to Figure 4, the example deep learning neural network model 400 is structured as follows according to this embodiment:
The example deep learning neural network model provides an end-to-end deep learning model for classifying emotional valence from (unimodal) heartbeat data. Recurrent and convolutional architectures are used to model temporal structure in the input signal.

A confidence value is output for each output (for example for each determined emotional state) from the model. In some examples, there is provided a procedure for tuning the model output depending on the threshold for acceptable certainty in the outputs from the model. In some examples, the model only outputs when the output is determined to have a confidence value above a predetermined confidence threshold (purely by way of example, above 50% or 70% or 90%). In applications of affective computing (i.e. automated emotion detection), this will be important in order to provide predictive interpretability for the model, for example in domains such as healthcare (where high certainty will be required, and so it is better not to output a classification with low certainty) or other domains (where a classification is needed, even if it only has a low certainty).

A number of known machine learning models only output a point estimate as a prediction, i.e. no confidence information for each point estimate is provided nor is any confidence value taken into account when outputting the point estimates. Typical examples of tasks using such known machine learning models include detecting pedestrians in images taken from an autonomous vehicle, classifying gene expression patterns from leukaemia patients into sub-types by clinical outcome or translating English sentences into French. In contrast, in the present embodiment, the confidence values of the outputs are output in order to be taken into account by a user or medical professional. In some examples, the confidence values of the outputs are taken into account by the model.

The example deep learning neural network model is structured in a sequence of layers: an input layer 410; a convolution layer 420; a Bidirectional Long Short-Term Memory Networks (BLSTM) layer 430; a concatenation layer 440; and an output layer 450.

The input layer 410 takes the information input into the network and causes it to flow to the next layers in the network, the convolution layer 420 and the BLSTM layer 430.

The convolution layer 420 consist of multiple hidden layers 421 , 422, 423, 424 (more than four layers may be present but these are not be shown in the Figure), the hidden layers typically consisting of one or any combination of convolutional layers, activation function layers, pooling layer, fully connected layers and normalisation layers.

There are many forms of uncertainty in modelling. At the lowest level, model uncertainty is introduced from measurement noise, e.g., pixel noise or blur in images. At higher levels, a model may have many parameters, such as the coefficients of a linear regression, and there is uncertainty about which values of these parameters will be good at predicting new data. Finally, at the highest levels, there is often uncertainty about even the general structure of the model.

Using a probabilistic framework in the described embodiment for machine learning allows modelling of these forms of uncertainty.

A Bayesian framework is used to model uncertainty in mental or emotional state predictions. Traditional neural networks can lack probabilistic interpretability, but this is an important issue in some domains such as healthcare. In an embodiment, neural networks are recast as Bayesian models to capture probability in the output. In this formalism, network weights belong to some prior distribution with parameters θ. Posterior distributions are then conditioned on the data according to Bayes' rule:

$$p(\theta|D) = \frac{p(D|\theta)p(\theta)}{p(D)}$$

(Equation 1)

where D is the data.

While useful from a theoretical perspective, Equation 1 is infeasible to compute. Instead, the posterior distributions can be approximated using a Monte-Carlo dropout method (alternatively embodiments can use methods including Monte Carlo or Laplace approximation methods, or stochastic gradient Langevin diffusion, or expectation propagation or variational methods). Dropout is a process by which individual nodes within the network are randomly removed during training according to a specified probability. By implementing dropout at test and performing N stochastic forward passes through the network, a posterior distribution can be approximated over model predictions (approaching the true distribution as N → ∞). In the embodiment, the Monte-Carlo dropout technique is implemented as an efficient way to describe uncertainty over mental or emotional state predictions.

In the described embodiments and aspects, probabilistic approaches to machine learning allow generation of a measure of confidence in the model's prediction. This can be critical for applications in healthcare where confidence and/or uncertainty is a key component of the downstream decision-making processes.

Examples of probabilistic models that can be used in alternative embodiments or in other aspects include (but are not limited to): directed graphical models, Markov chains, Gaussian processes, and even probabilistic approximations of deep neural networks (often referred to as Bayesian neural networks). In the described embodiment a Bayesian neural network is used for predicting mental states/emotion data from heartbeat data.

A benefit of the probabilistic approach to machine learning of the present embodiment can be that it provides a meaningful way to deal with limited data available when using data from, for example, mobile and wearable devices. This is in contrast with traditional non-probabilistic deep learning, which requires significant amounts of data in comparison.

The BLSTM layer 430 is a form of generative deep learning where two hidden layers 431 , 432 of opposite directions are connected to the same output to get information from past (the "backwards" direction layer) and future (the "forwards" direction layer) states simultaneously. The layer 430 functions to increase the amount of input information available to the network 400, and provide the functionality of providing context for the input layer 410 information (i.e. data/inputs before and after, temporally, the current data/input being processed).

The concatenation layer 440 concatenates the output from the convolution layer 420 and the BLSTM layer 430.

The output layer 450 then outputs the final result 451 for the input 410, dependent on whether the output layer 450 is designed for regression or classification. If the output layer 450 is designed for regression, the final result 451 is a regression output of continuous emotional valence and/or arousal. If the output layer 450 is designed for classification, the final result 451 is a classification output, i.e. a discrete mental state and/or emotional state.

Data flows through two concurrent streams in the model 400. One stream comprises four stacked convolutional layers that extract local patterns along the length of the time series. Each convolutional layer is followed by dropout and a rectified linear unit activation function (i.e. converting the output to a 0 or a 1). A global average pooling layer is then applied to reduce the number of parameters in the model and decrease over-fitting. The second stream comprises a bidirectional LSTM followed by dropout. This models both past and future sequence structure in the input. The output of both streams are then concatenated before passing through a dense layer to output a regression estimate for valence.

In order to capture uncertainty in the model predictions, dropout is applied at test time. For a single input sample, stochastic forward propagation is run N times to generate a distribution of model outputs. This empirical distribution approximates the posterior probability over valence given the input time series. At this point, a regression output can be generated by the model.

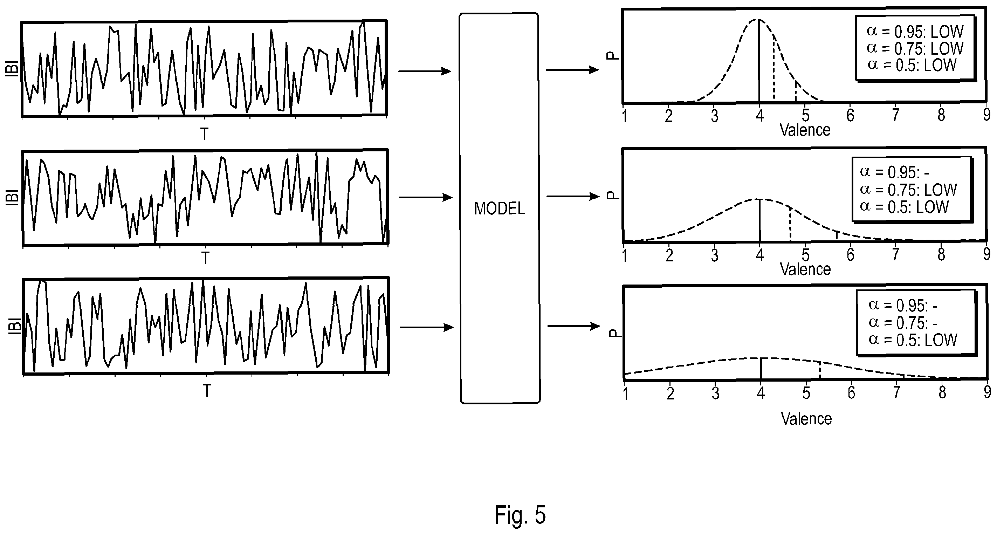

To generate a classification output, i.e. to translate from a regression to a classification scheme, decision boundaries in continuous space need to be introduced. For a binary class problem, this decision boundary is along the central point of the valence scale to delimit two class zones (high and low valence for example). Next a confidence threshold parameter $\alpha$ is used to tune predictions to a specified level of model uncertainty. For example, when $\alpha = 0.95$, at least 95% of the output distribution must lie in a given class zone in order for the input sample to be classified as belonging to that class (see Figure 5). If this is not the case, then no prediction is made. The model may therefore not classify all instances so the model only outputs a classification when the threshold that has been predetermined is met. As $\alpha$ increases, the model behaviour moves from risky to cautious but with less likelihood that a classification will be output (but with more certainty for classifications that are output). For binary classifications, there will always be at least 50% of the output distribution that will be within one of the two prediction zones, thus when $\alpha = 0.5$ the classification is determined by the median of the output distribution and a classification will always be made.

In other embodiments, variations of this network structure are possible but require the deep neural network model to model time dependency such that it uses the previous state of the network along, and/or temporal information within the input signal, to output a valence score. Other neural network structures can be used.

The training process for the model in the embodiment works as follows:

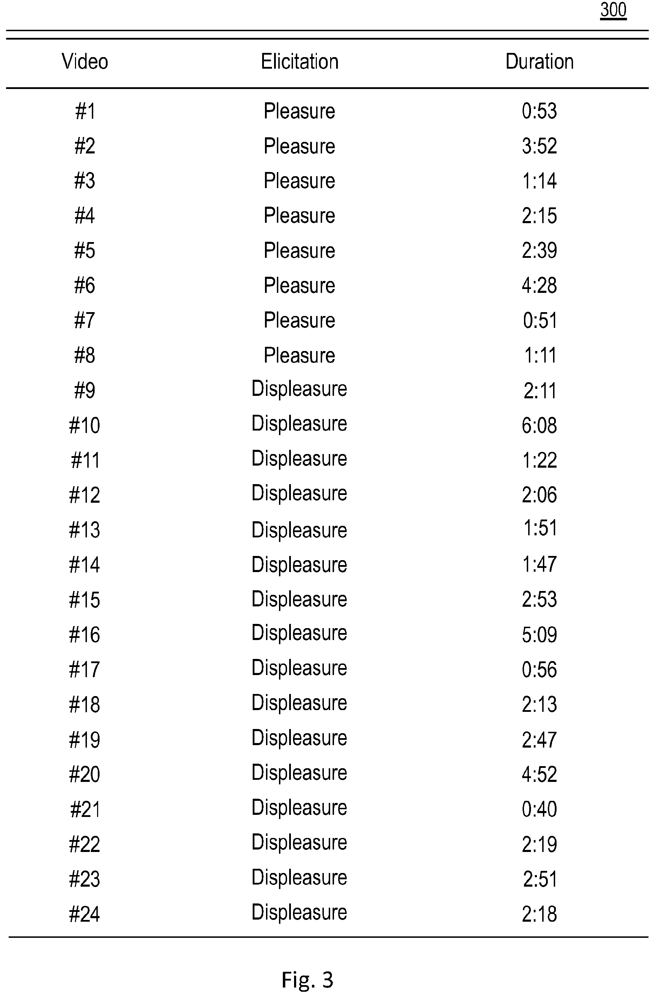

Referring to Figure 3, users wearing a wearable device such as the smartwatch 100 are exposed to emotion-eliciting stimuli (e.g. video stimuli #1 to #24) that has been scored independently for its ability to induce both pleasurable and displeasurable feelings in viewers ("Elicitation"). The table 300 in Figure 3 shows a table of 24 example video stimuli along with an associated pleasure/displeasure rating for each video and a length of each video.

In the embodiment where the stimuli are video stimuli, each user watches the series of videos and, after each video, each user is asked to rate their own mental state for pleasure and displeasure in line with the "valence" metric from the psychological frameworks for measuring emotion (e.g. the popular self-assessment Manikin (SAM) framework). A statistically significant sample size of users will be needed. Additionally, a one-minute neutral video following each user completing their rating of their mental state should allow the user to return to a neutral mental state between viewing the next emotion-eliciting video. Further, playing the video sequence in a different random order to each user should improve the training process.

It will be understood that other options for stimuli are possible to carry out this process. In some embodiments, other options for training are possible in order to collect input-output pairs, where the input data is a physiological data time series and the output data (to which the input data is paired) is user mental state (this data can be self- reported/explicit or inferred from analysing users using text and/or facial data and/or speech or other user data).

Referring to Figure 4, and once the model has been training, a standalone output model is produced that can be deployed on a wearable device to predict the mental and/or emotional state of a user of the wearable device on which the model is deployed. Additionally, the model is able to predict the mental and/or emotional state of a user even where the specific input data hasn't been seen in the training process. The predicted mental or emotional state is output with a confidence level by the model. Bayesian neural network architectures can be used in some embodiments to model uncertainty in the model parameters and the model predictions. In other embodiments, probabilistic models capable of describing uncertainty in their output can be used.

Once the model is deployed on the wearable device, further functionality can be enabled for users.

As described above, other types of learned algorithm can be used apart from that described in the embodiments.

In some embodiments, the learned algorithms can be configured to determine both the current and previous mental states of a user wearing the wearable device, typically by providing local storage on the wearable device and saving mental state data on the local storage (or uploading part or all of this data to remote storage).

The learned algorithm also outputs confidence data for the determined mental state of the user of the wearable device, as sometimes it will be highly probable that a user is in a particular mental state given a set of inputs but in other situations the set of inputs will perhaps only give rise to a borderline determination of an mental state, in which case the output of the algorithm will be the determined mental state but with a probability reflecting the level of uncertainty that this is the correct determined mental state.

All suitable types of format of wearable device are intended to be usable in embodiments, but in alternative embodiments other suitable devices can be used such as smartphones, provided that the device has sufficient hardware and software capabilities to perform the computation required and be configured to operate the software to perform the embodiments and/or alternatives described herein. For example, in some embodiments the device could be any of a smartwatch; a wearable sensor; a fitness band; smart ring; headset; smart textile; wearable patch; or smartphones. Other wearable device formats will also be appropriate, as it will be apparent.

In some embodiments, should the device have location determination capabilities, for example using satellite positioning or triangulation based on cell-towers or Wi-Fi access points, then the location of the device can be associated with the user's mental or emotional state and a geographic map of mental or emotional states determined. As an example, one or more users may consistently experience certain mental or emotional states in certain locations (for example famous landmarks, or at their homes or places of work). This type of emotion-location association can be anonymised and used for various purposes aggregated over a number of users, or users can view and use their own emotion-location associations for their own purposes.

In some embodiments, some of the processing to use the model can be done remotely and/or the model/learned algorithm can be updated remotely and the model on the user device can be updated with the version that has been improved and which is stored remotely. Typically, some form of software updating process run locally on the user device will poll a remote computer which will indicate that a newer model is available and allow the user device to download the updated model and replace the locally-stored model with the newly downloaded updated model. In some embodiments, data from the user device will be shared with one or more remote servers to enable the model(s) to be updated based on one or a plurality of user data collected by user devices.

In some embodiments, the mental states being determined include any or any combination of discrete emotions such as: depression; happiness; pleasure; displeasure; and/or dimensional emotions such as arousal and valence.

In some embodiments, establishing or determining a link between the mental state of the user and other factors related to the user is done using the user device such as a wearable device worn by the user. The other factors can be any or any combination of: an activity level of the user; an exercise measure of the user and/or a lifestyle measure of the user over a period of time and determining a correlation between the current mental state of the user and any or any combination of: the activity level of the user; the exercise measure of the user and/or the lifestyle measure of the user. It is generally accepted that the activity, exercise and/or lifestyle of people are correlated with their mental state and the use of the detected mental state of the user and data on a user's activity, exercise and/or lifestyle will allow correlations to be determined. Optionally, the user device and/or a linked system such as a phone application or other computer software can then notify the user based on any determined correlations, for example a user might be showing signs that they are depressed or sad, and that user may have a strong correlation between doing exercise and feeling happier, so a notification might be some form of nudge or recommendation to that user to do some exercise soon so that they feel happier.

The user attribute data is data from a photoplethysmography time series. In some embodiments, data from a variety of sensors can be used to determine a mental state and/or emotional state, including any or any combination of: face imaging data; image and/or text data; speech data; electrodermal activity data; electrocardiogram data; respiration data; temperature data; gyroscope data; wearer activity data and/or accelerometer data.

In some embodiments, a variety of devices can be used, alone or in combination, to determine the mental state and/or emotional state of a user including any or any combination of: a mobile phone; a computer; a wearable device; an imaging camera; an audio sensor; an audiovisual device; a smartwatch; a wearable sensor; a fitness band; a smart ring, a smart textile, a headset or a wearable patch.

In some embodiments, users may want to send details of their current mental state to third parties, such as friends, family or even medical professionals. The details that a user might send may be an emoticon closely correlated with what their user device is currently detecting as their mental state or may be a full dataset and a spectrum of levels of data in between these simple and complex options for transmitting these types of details to third parties.

Any system feature as described herein may also be provided as a method feature, and vice versa. As used herein, means plus function features may be expressed alternatively in terms of their corresponding structure.

Any feature in one aspect of the invention may be applied to other aspects, in any appropriate combination. In particular, method aspects may be applied to system aspects, and vice versa. Furthermore, any, some and/or all features in one aspect can be applied to any, some and/or all features in any other aspect, in any appropriate combination.

It should also be appreciated that particular combinations of the various features described and defined in any aspects can be implemented and/or supplied and/or used independently.

**Claims**

1. A method to determine a mental state of a user, comprising:

   receiving user attribute data from one or more sensors, wherein the user attribute data is data from a photoplethysmography time series; determining the mental state of the user from the user attribute data using a learned neural network model, the learned neural network model comprising an input layer (410), a convolution layer (420), a bidirectional long short-term memory BLSTM layer (430), a concatenation layer (440) and an output layer (450), wherein the input layer (410) takes data input to the learned neural network model and causes it to flow to the convolution layer (420) and the BLSTM layer (430), and wherein the concatenation layer (440) concatenates the output from the convolution layer (420) and the BLSTM layer (430);
outputting one or more determined mental states for the user; and
outputting one or more confidence values of the one or more determined mental states of the user.

2. The method of claim 1, wherein the mental state of the user comprises any or any combination of: an emotional state; a plurality of emotional states; one or more discrete emotional states; one or more continuous emotional states; one or more discrete or continuous measures of emotion; one or more psychological states; one or more psychological states linked with mental illness; one or more psychological states comprising any or any combination of depression, anxiety, bipolar disorder and psychosis; discrete emotions such as depression, happiness, pleasure, displeasure; and/or dimensional emotions such as arousal and valence.

3. The method of any preceding claim, wherein:

   receiving user attribute data from the one or more sensors comprises receiving user attribute data from the one or more sensors substantially continuously;
   determining a mental state of the user from the user attribute data comprises substantially continuously determining a mental state of the user from the user attribute data; and
   outputting one or more determined mental states for the user comprises outputting one or more substantially continuously determined mental states for the user.

4. The method of any preceding claim, further comprising outputting user attribute data corresponding to the determined mental states of the user for use by a medical professional.

5. The method of any preceding claim, further including:

   associating the determined mental state of the user with a location of the user; and
   storing each associated mental state of the user and associated location of the user in a database.

6. The method of claim 5, wherein the database is a local database or a remote database.

7. The method of claim 5 or 6, wherein the sensor data includes user location data.

8. The method of any preceding claim, wherein the learned neural network model is operable to be updated by a remote computing system.

9. The method of claim 8, wherein the learned neural network model is operable to be updated by the remote computing system using some or all of the received user attribute data and/or the determined user mental states; and/or the remote computing system is able to perform at least a portion of the step of determining the mental state of the user.

10. The method of any preceding claim, further comprising receiving any or any combination of: an activity level of the user; an exercise measure of the user and/or a lifestyle measure of the user over a period of time and determining a correlation between the determined mental state of the user and any or any combination of: the activity level of the user; the exercise measure of the user and/or the lifestyle measure of the user; optionally generating a notification for display to a user in response to the determined correlation.

11. A computer program product operable to provide the method of any preceding claim.

12. An apparatus operable to provide the method of any of claims 1 to 10.

13. The apparatus of claim 12, wherein the apparatus comprises any of a wearable device; a smartwatch; a wearable sensor; a fitness band; a smart ring, a smart textile, a headset or a wearable patch.

14. A wearable apparatus comprising one or more sensors and configured to determine the mental state of a wearer, the apparatus operable to perform the steps of:

   receiving wearer physiological data from the one or more sensors, wherein the wearer physiological data is data from a photoplethysmography time series;
   determining a mental state of the wearer from the wearer physiological data using a learned neural network model, the learned neural network model comprising an input layer (410), a convolution layer (420), a bidirectional long short-term memory BLSTM layer (430), a concatenation layer (440) and an output layer (450), wherein the input layer (410) takes data input to the learned neural network model and causes it to flow to the convolution layer (420) and the BLSTM layer (430), and wherein the concatenation layer (440) concatenates the output from the convolution layer (420) and the BLSTM layer (430);

outputting one or more determined mental states for the wearer; and

outputting a confidence value of the one or more determined mental states of the wearer.

15. The wearable apparatus of claim 14, wherein the mental state of the wearer comprises the current mental state of the wearer.

**Patentansprüche**

1. Verfahren zum Feststellen eines mentalen Zustandes eines Benutzers, das Folgendes umfasst:

Empfangen von Benutzerattributdaten von einem oder mehreren Sensoren, wobei die Benutzerattributdaten Daten aus einer Photoplethysmographie-Zeitreihe sind,

Feststellen des mentalen Zustandes des Benutzers aus den Benutzerattributdaten unter Verwendung eines trainierten Neuronalnetzmodells, wobei das trainierte Neuronalnetzmodell eine Eingabeschicht (410), eine Faltungsschicht (420), eine bidirektionale Kurzzeitspeicher- , BLSTM-, Schicht (430), eine Verkettungsschicht (440) und eine Ausgabeschicht (450) umfasst, wobei die Eingabeschicht (410) in das trainierte neuronale Netzwerkmodell eingegebene Daten aufnimmt und veranlasst, dass sie zu der Faltungsschicht (420) und der BLSTM-Schicht (430) fließen, und wobei die Verkettungsschicht (440) die Ausgabe aus der Faltungsschicht (420) und der BLSTM-Schicht (430) verkettet,

Ausgeben eines oder mehrerer festgestellter mentaler Zustände für den Benutzer und Ausgeben eines oder mehrerer Vertrauenswerte des einen oder der mehreren festgestellten mentalen Zustände des Benutzers.

2. Verfahren nach Anspruch 1, wobei der mentale Zustand des Benutzers ein beliebiges oder eine beliebige Kombination von Folgendem umfasst: einen emotionalen Zustand, eine Vielzahl von emotionalen Zuständen, einen oder mehrere diskrete emotionale Zustände, einen oder mehrere kontinuierliche emotionale Zustände, ein oder mehrere diskrete oder kontinuierliche Emotionsausmaße, einen oder mehrere psychologische Zustände, einen oder mehrere mit psychischer Erkrankung verbundene psychologische Zustände, einen oder mehrere psychologische Zustände, die ein beliebiges oder eine beliebige Kombination von Depression, Angst, bipolarer Störung und Psychose umfassen, diskrete Emotionen wie beispielsweise Depression, Glück, Vergnügen, Missvergnügen und/oder dimensionale Emotionen wie beispielsweise Erregung und Valenz.

3. Verfahren nach einem der vorhergehenden Ansprüche, wobei:

das Empfangen von Benutzerattributdaten von dem einen oder den mehreren Sensoren das im Wesentlichen kontinuierliche Empfangen von Benutzerattributdaten von dem einen oder den mehreren Sensoren umfasst, das Feststellen eines mentalen Zustandes des Benutzers aus den Benutzerattributdaten das im Wesentlichen kontinuierliche Feststellen eines mentalen Zustandes des Benutzers aus den Benutzerattributdaten umfasst und das Ausgeben eines oder mehrerer festgestellter mentaler Zustände für den Benutzer das Ausgeben eines oder mehrerer im Wesentlichen kontinuierlich festgestellter mentaler Zustände für den Benutzer umfasst.

4. Verfahren nach einem der vorhergehenden Ansprüche, das ferner das Ausgeben von Benutzerattributdaten, die den festgestellten mentalen Zuständen des Benutzers entsprechen, zur Verwendung durch eine medizinische Fachkraft umfasst.

5. Verfahren nach einem der vorhergehenden Ansprüche, das ferner Folgendes einschließt:

Verknüpfen des festgestellten mentalen Zustandes des Benutzers mit einem Standort des Benutzers und

Speichern jedes verknüpften mentalen Zustandes des Benutzers und des verknüpften Standorts des Benutzers in einer Datenbank.

6. Verfahren nach Anspruch 5, wobei die Datenbank eine örtliche Datenbank oder eine entfernte Datenbank ist.

7. Verfahren nach Anspruch 5 oder 6, wobei die Sensordaten Benutzerstandortdaten einschließen.

8. Verfahren nach einem der vorhergehenden Ansprüche, wobei das trainierte Neuronalnetzmodell funktionsfähig ist, um durch ein entferntes Datenverarbeitungssystem aktualisiert zu werden.

9. Verfahren nach Anspruch 8, wobei das trainierte Neuronalnetzmodell funktionsfähig ist, um unter Verwendung einiger oder aller der empfangenen Benutzerattributdaten und/oder der festgestellten mentalen Benutzerzustände durch das entfernte Datenverarbeitungssystem aktualisiert zu werden, und/oder das entfernte Datenverarbeitungssystem dazu in der Lage ist, mindestens einen Teil des Schritts des Feststellens des mentalen Zustandes des Benutzers durchzuführen.

10. Verfahren nach einem der vorhergehenden Ansprüche, das ferner das Empfangen eines beliebigen oder einer beliebigen Kombination von Folgendem: eines Aktivitätsniveaus des Benutzers, eines Übungsausmaßes des Benutzers und/oder eines Lebensstilmaßes des Benutzers über einen Zeitraum und das Feststellen einer Korrelation zwischen dem mentalen Zustand des Benutzers und eines beliebigen oder einer beliebigen Kombination von dem Aktivitätsniveau des Benutzers, dem Übungsausmaß des Benutzers und/oder dem Lebensstilmaß des Benutzers, wahlweise das Erzeugen einer Benachrichtigung zur Anzeige für einen Benutzer als Reaktion auf die festgestellte Korrelation umfasst.

11. Rechnerprogrammerzeugnis, das funktionsfähig ist, um das Verfahren nach einem der vorhergehenden Ansprüche bereitzustellen.

12. Vorrichtung, die funktionsfähig ist, um das Verfahren nach einem der Ansprüche 1 bis 10 bereitzustellen.

13. Vorrichtung nach Anspruch 12, wobei die Vorrichtung eines von einem tragbaren Gerät, einer Smartwatch, einem tragbaren Sensor, einem Fitnessband, einem Smartring, einem smarten Textil, einer Sprechgarnitur oder einem tragbaren Aufnäher umfasst.

14. Tragbare Vorrichtung, die einen oder mehrere Sensoren umfasst und dafür konfiguriert ist, den mentalen Zustand eines Trägers festzustellen, wobei die Vorrichtung funktionsfähig ist, um die folgenden Schritte durchzuführen:

Empfangen von physiologischen Trägerdaten von einem oder mehreren Sensoren, wobei die physiologischen Trägerdaten Daten aus einer Photoplethysmographie-Zeitreihe sind, Feststellen eines mentalen Zustandes des Benutzers aus den physiologischen Trägerdaten unter Verwendung eines trainierten Neuronalnetzmodells, wobei das trainierte Neuronalnetzmodell eine Eingabeschicht (410), eine Faltungsschicht (420), eine bidirektionale Kurzzeitspeicher- , BLSTM-, Schicht (430), eine Verkettungsschicht (440) und eine Ausgabeschicht (450) umfasst, wobei die Eingabeschicht (410) in das trainierte neuronale Netzwerkmodell eingegebene Daten aufnimmt und veranlasst, dass sie zu der Faltungsschicht (420) und der BLSTM-Schicht (430) fließen, und wobei die Verkettungsschicht (440) die Ausgabe aus der Faltungsschicht (420) und der BLSTM-Schicht (430) verkettet,
Ausgeben eines oder mehrerer festgestellter mentaler Zustände für den Träger und
Ausgeben eines Vertrauenswertes des einen oder der mehreren festgestellten mentalen Zustände des Trägers.

15. Tragbare Vorrichtung nach Anspruch 14, wobei der mentale Zustand des Trägers den gegenwärtigen mentalen Zustand des Trägers umfasst.


**Revendications**

1. Procédé de détermination d'un état mental d'un utilisateur, comprenant les étapes suivantes :

réception de données d'attributs d'utilisateur à partir d'un ou de plusieurs capteurs, dans lequel les données d'attributs d'utilisateur sont des données provenant d'une série chronologique de photopléthysmographie ;
détermination de l'état mental de l'utilisateur à partir des données d'attributs d'utilisateur en utilisant un modèle de réseau neuronal appris, le modèle de réseau neuronal appris comprenant une couche d'entrée (410), une couche de convolution (420), une couche de mémoire bidirectionnelle à long-court terme, BLSTM (430), une couche de concaténation (440) et une couche de sortie (450), dans lequel la couche d'entrée (410) prend des données entrées dans le modèle de réseau neuronal appris et les fait circuler vers la couche de convolution (420) et la couche BLSTM (430), et dans lequel la couche de concaténation (440) concatène la sortie de la couche de convolution (420) et de la couche BLSTM (430) ;
sortie d'un ou de plusieurs états mentaux déterminés pour l'utilisateur ; et
sortie d'une ou de plusieurs valeurs de confiance desdits un ou plusieurs états mentaux déterminés de l'utilisateur.

2. Procédé selon la revendication 1, dans lequel l'état mental de l'utilisateur comprend un quelconque ou une quelconque combinaison des éléments suivants : un état émotionnel, une pluralité d'états émotionnels, un ou plusieurs états émotionnels discrets, un ou plusieurs états émotionnels continus ; une ou plusieurs mesures discrètes ou continues d'émotion ; un ou plusieurs états psychologiques ; un ou plusieurs états psychologiques liés à une maladie mentale ; un ou plusieurs états psychologique comprenant un quelconque ou une quelconque combinaison d'une dépression, d'une anxiété, d'un trouble bipolaire et d'une psychose, des émotions discrètes telles que la dépression, le bonheur, le plaisir, le déplaisir et/ou des émotions dimensionnelles telles que l'excitation et la valence.

3. Procédé selon l'une quelconque des revendications précédentes, dans lequel :

la réception des données d'attributs d'utilisateur provenant des un ou plusieurs capteurs comprend la réception de données d'attributs d'utilisateur provenant des un ou plusieurs capteurs de manière sensiblement continue ;
la détermination d'un état mental de l'utilisateur sur la base des données d'attributs d'utilisateur comprend la détermination sensiblement continue d'un état mental de l'utilisateur sur la base des données d'attributs d'utilisateur ; et
l'émission en sortie d'un ou de plusieurs états mentaux déterminés pour l'utilisateur comprend la sortie d'un ou de plusieurs états mentaux pour l'utilisateur déterminés de manière sensiblement continue.

4. Procédé selon l'une quelconque des revendications précédentes, comprenant en outre l'étape de sortie de données d'attributs d'utilisateur correspondant aux états mentaux déterminés de l'utilisateur en vue d'un utilisation par un professionnel de la santé.

5. Procédé selon l'une quelconque des revendications précédentes, incluant en outre les étapes suivantes :

association de l'état mental déterminé de l'utilisateur à un emplacement de l'utilisateur ; et stockage de chaque état mental associé de l'utilisateur et de l'emplacement associé de l'utilisateur dans une base de données.

6. Procédé selon la revendication 5, dans lequel la base de données est une base de données locale ou une base de données à distance.

7. Procédé selon les revendications 5 ou 6, dans lequel les données de capteur incluent des données d'emplacement de l'utilisateur.

8. Procédé selon l'une quelconque des revendications précédentes, dans lequel le modèle de réseau neuronal appris est exploitable pour être mis à jour par un système informatique à distance.

9. Procédé selon la revendication 8, dans lequel le modèle de réseau neuronal appris est exploitable pour être mis à jour par le système informatique à distance en utilisant certaines des ou toutes les données d'attributs d'utilisateur reçues et/ou les états mentaux déterminés de l'utilisateur ; et/ou le système informatique à distance peut exécuter au moins une partie de l'étape de détermination de l'état mental de l'utilisateur.

10. Procédé selon l'une quelconque des revendications précédentes, comprenant en outre les étapes de réception d'un quelconque ou d'une quelconque combinaison des éléments suivants : un niveau d'activité de l'utilisateur ; une mesure de l'exercice de l'utilisateur et/ou une mesure du style de vie de l'utilisateur sur une période de temps et détermination d'une corrélation entre l'état mental déterminé de l'utilisateur et un quelconque ou une combinaison des éléments suivants : le niveau d'activité de l'utilisateur ; la mesure de l'exercice de l'utilisateur et/ou la mesure du style de vie de l'utilisateur ; et optionnellement, de génération d'une notification à afficher à un utilisateur en réponse à la corrélation déterminée.

11. Produit-programme informatique exploitable pour fournir le procédé selon l'une quelconque des revendications précédentes.

12. Appareil utilisable pour fournir le procédé selon l'une quelconque des revendications 1 à 10.

13. Appareil selon la revendication 12, dans lequel l'appareil comprend un quelconque des éléments suivants : un dispositif portable ; une montre intelligente ; un capteur portable ; un bracelet de fitness ; une bague intelligente : un textile intelligent, un casque ou un patch portable.

14. Appareil portable, comprenant un ou plusieurs capteurs et configuré pour déterminer l'état mental d'un utilisateur, l'appareil étant exploitable pour effectuer les opérations suivantes :

réception de données physiologiques de l'utilisateur provenant d'un ou de plusieurs capteurs, dans lequel les données physiologiques de l'utilisateur sont des données provenant d'une série chronologique de photopléthysmographie ;
détermination d'un état mental de l'utilisateur à partir des données physiologiques du porteur en utilisant un modèle de réseau neuronal appris, le modèle de réseau neuronal appris comprenant une couche d'entrée (410), une couche de convolution (420), une couche de mémoire longue à court terme bidirectionnelle BLSTM (430), une couche de concaténation (440) et une couche de sortie (450), dans lequel la couche d'entrée (410) prend des données entrées dans le modèle de réseau neuronal appris et les fait circuler vers la couche de convolution (420) et la couche BLSTM (430), et dans lequel la couche de concaténation (440) concatène la sortie de la couche de convolution (420) et de la couche BLSTM (430) ;

sortie d'un ou de plusieurs états mentaux déterminés pour l'utilisateur ; et

sortie d'une ou de plusieurs valeurs de confiance des un ou plusieurs états mentaux déterminés de l'utilisateur.

15. Appareil portable selon la revendication 14, dans lequel l'état mental de l'utilisateur comprend l'état mental actuel du porteur.

Fig. 1

Fig. 2

| Video | Elicitation | Duration |
|---|---|---|
| #1 | Pleasure | 0:53 |
| #2 | Pleasure | 3:52 |
| #3 | Pleasure | 1:14 |
| #4 | Pleasure | 2:15 |
| #5 | Pleasure | 2:39 |
| #6 | Pleasure | 4:28 |
| #7 | Pleasure | 0:51 |
| #8 | Pleasure | 1:11 |
| #9 | Displeasure | 2:11 |
| #10 | Displeasure | 6:08 |
| #11 | Displeasure | 1:22 |
| #12 | Displeasure | 2:06 |
| #13 | Displeasure | 1:51 |
| #14 | Displeasure | 1:47 |
| #15 | Displeasure | 2:53 |
| #16 | Displeasure | 5:09 |
| #17 | Displeasure | 0:56 |
| #18 | Displeasure | 2:13 |
| #19 | Displeasure | 2:47 |
| #20 | Displeasure | 4:52 |
| #21 | Displeasure | 0:40 |
| #22 | Displeasure | 2:19 |
| #23 | Displeasure | 2:51 |
| #24 | Displeasure | 2:18 |

Fig. 3

Fig. 4

EP 3 917 400 B1

18

Fig. 5

EP 3 917 400 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018032126 A1, RINCON JAIME A
- US 20181109 A1
- US 9566411 B1

**Non-patent literature cited in the description**

- ADVANCES IN DATABASES AND INFORMATION SYSTEMS; [LECTURE NOTES IN COMPUTER SCIENCE; LECT.NOTES COMPUTER. SPRINGER INTERNATIONAL PUBLISHING, 520-530